# EUROPÄISCHE PATENTSCHRIFT

(11) **EP 0 707 002 B1**
(45) Veröffentlichungstag und Bekanntmachung des Hinweises auf die Patenterteilung: **26.03.2003**
(21) Anmeldenummer: 95810625.4
(22) Anmeldetag: 04.10.1995
(51) Int. Cl.: C07D 251/50, C07D 251/52, C07D 239/48, C07D 239/50, C07C 233/43, D06M 13/358

(54) **UV-Absorber, ihre Herstellung und Verwendung**
UV absorbers, their preparation and use
Absorbants UV, leur préparation et leur application

(30) Priorität: 13.10.1994 CH 308094
(43) Veröffentlichungstag der Anmeldung: 17.04.1996
(73) Patentinhaber: Ciba Specialty Chemicals Holding Inc., 4057 Basel (CH)
(72) Erfinder: Fuso, Francesco, Dr., CH-4106 Therwil (CH); Reinert, Gerhard, Dr., CH-4123 Allschwil (CH)

(56) Entgegenhaltungen:
- EP-A- 0 388 356
- EP-A- 0 552 605
- WO-A-94/04515
- DE-A- 1 568 351
- DE-A- 3 740 650
- DE-B- 1 618 196
- DE-B- 1 618 197
- US-A- 3 041 330
- US-A- 3 906 033
- US-A- 3 906 041

## Beschreibung

Gegenstand der vorliegenden Erfindung sind neue faserreaktive UV-Absorber, Verfahren zu ihrer Herstellung und ihre Verwendung zur photochemischen Stabilisierung von ungefärbten und gefärbten Textilfasern und zur Verbesserung des Sonnenschutzfaktors von solchen Textilfasern.

Die hautschädigende Wirkung von UV-Strahlung ist bekannt. Üblicherweise schützt man sich vor starker Sonnenbestrahlung, indem man eine UV-Absorber enthaltende Zusammensetzung (Sonnencreme) direkt auf die Haut aufgeträgt. In besonders sonnigen Bereichen der Erde, z.B. in Australien oder Amerika, steigt jedoch die Rate der durch UV-Strahlung bedingten Hautschädigungen in neuerer Zeit drastisch an. Dementsprechend wird dem Schutz der Haut vor Sonnenbestrahlung in diesen Ländern eine erhöhte Aufmerksamkeit geschenkt.

Es ist daher vorgeschlagen worden, nicht nur die Haut direkt, sondern auch die sie umgebende Bekleidung sowie textile Sonnenschutzartikel wie Markisen oder Sonnenschirme zusätzlich gegen UV-Strahlung zu schützen. Die meisten natürlichen und synthetischen Textilmaterialien, ob ungefärbt oder gefärbt, sind nämlich zumindest teilweise durchlässig für UV-Strahlung, sodass das blosse Tragen von Bekleidung keinen angemessenen Schutz der Haut vor Schädigungen durch UV-Strahlung bietet. Abhilfe ist hier möglich durch die Einarbeitung von UV-Absorbern in das Textilgewebe.

Die EP-A-0 388 356 sowie die EP-A-0 552 605 offenbaren UV-Absorber auf der Basis von Oxalsäurediamiden mit reaktiven Halogentriazin- bzw. Vinylsulfonylgruppen.

Die im Bereich der Textilmaterialien, insbesondere der Cellulosefasern oder natürliche oder synthetische Polyamidfasern enthaltenden Textilmaterialien, erzielten Ergebnisse bezüglich Schutz vor UV-Strahlung sind jedoch bisher noch nicht befriedigend, und es besteht ein Bedürfnis, neue speziell auf diese Materialien abgestimmte UV-Absorber zu entwickeln.

Gegenstand der vorliegenden Erfindung sind daher Verbindungen der Formel 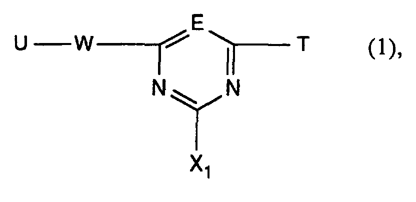 worin
U für den Rest eines Oxalsäurediamids der Formel 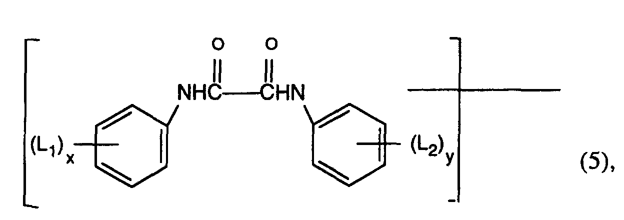 steht, worin (L₁)ₓ für x gleiche oder verschiedene Substituenten L₁ aus der Gruppe Sulfo, unsubstituiertes oder im Alkylteil durch Sulfo substituiertes Alkyl, Alkoxy oder Alkylthio mit je 1 bis 22 Kohlenstoffatomen und unsubstituiertes oder im Phenylring durch Sulfo substituiertes Phenoxy oder Phenylthio steht, (L₂)_{y} y gleiche oder verschiedene Substituenten L₂ aus der Gruppe Sulfo, unsubstituiertes oder im Alkylteil durch Sulfo substituiertes Alkyl, Alkoxy oder Alkylthio mit je 1 bis 22 Kohlenstoffatomen und unsubstituiertes oder im Phenylring durch Sulfo substituiertes Phenoxy oder Phenylthio bedeutet, und x und y unabhängig voneinander je eine ganze Zahl von 0 bis 3 bedeuten, wobei die Summe von (x + y) ≥ 1 ist,
W eine Gruppe -NR₂- bedeutet,
R₂ für Wasserstoff oder unsubstituiertes oder durch Halogen, Hydroxy, Cyano, C₁-C₄-Alkoxy, C₁-C₄-Alkoxycarbonyl, Carboxy, Sulfamoyl, Sulfo oder Sulfato substituiertes C₁-C₄-Alkyl steht,
=E- für eine Gruppe =N- oder =C(T₁)- steht und T₁ Halogen, C₁-C₄-Alkylsulfonyl, Formyl, C₂-C₄-Alkoxycarbonyl oder Cyano ist,
X₁ Halogen, Hydroxy, Sulfo, C₁-C₄-Alkylsulfonyl, Phenylsulfonyl, Amino, unsubstituiertes oder im Alkylteil durch Hydroxy, Carboxy, Sulfo, Sulfato oder C₁-C₄-Alkoxy substituiertes N-Mono- oder N,N-Di-C₁-C₄-Alkylamino, Cyclohexylamino, unsubstituiertes oder im Phenylteil durch C₁-C₄-Alkyl, C₁-C₄-Alkoxy, Carboxy, Sulfo, Halogen oder durch einen Rest der Formel

-SO₂-Y (6a),

-CONH-(CH₂)ₚ-SO₂-Y (6b),

worin p eine ganze Zahl 1 bis 6 bedeutet, substituiertes Phenylamino oder N-C₁-C₄-Alkyl-N-phenylamino, einfach oder mehrfach durch Sulfo substituiertes 1- oder 2-Naphthylamino, 3-Carboxypyridin-1-yl oder 3-Carbamoylpyridin-1-yl bedeutet, T ein Reaktivrest der Formel 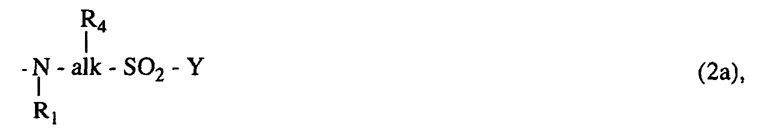 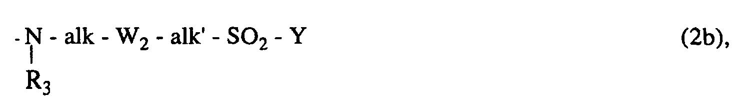 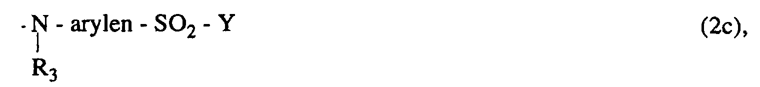 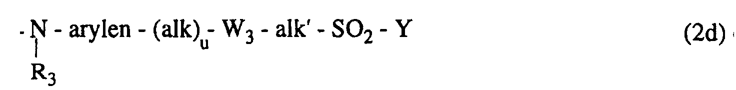 oder 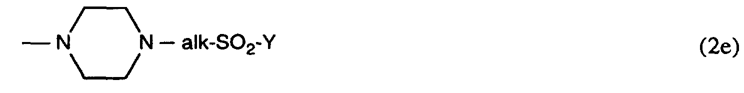 ist,
R₁ Wasserstoff, gegebenenfalls durch Hydroxy, Sulfo, Sulfato, Carboxy oder Cyano substituiertes C₁-C₄-Alkyl oder einen Rest 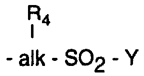 bedeutet, R₃ für Wasserstoff oder C₁-C₄-Alkyl steht,
R₄ Wasserstoff, Hydroxy, Sulfo, Sulfato, Carboxy, Cyano, Halogen, C₁-C₄-Alkoxycarbonyl, C₁-C₄-Alkanoyloxy, Carbamoyl oder die Gruppe -SO₂-Y ist, alk und alk' unabhängig voneinander C₁-C₆-Alkylen sind,
arylen einen unsubstituierten oder durch Sulfo, Carboxy, C₁-C₄-Alkyl, C₁-C₄-Alkoxy oder Halogen substituierten Phenylen- oder Naphthylenrest bedeutet
Y Vinyl oder ein Rest -CH₂-CH₂-Z und Z eine Gruppe -Cl, -Br, -F, -OSO₃H, -SSO₃H, -OCO-CH₃, -OPO₃H₂, -OCO-CCl₃, -OCO-CHCl₂, -OCO-CH₂Cl, -OSO₂-C₁-C₄-Alkyl, -OSO₂-N(C₁-C₄-Alkyl)₂ oder -OCO-C₆H₅ ist,
W₂ den Rest -O- oder -NR₃- bedeutet,
W₃ für eine Gruppe -SO₂-NR₁-, -CONR₁- oder -NR₁CO- steht,
u die Zahl 0 oder 1 bedeutet, mit der Massgabe, dass die Verbindungen der Formel (1) mindestens eine Sulfo- oder Sulfatogruppe aufweisen.

Vorzugsweise bedeutet R₂ Wasserstoff oder C₁-C₄-Alkyl und insbesondere bevorzugt Wasserstoff, Methyl oder Ethyl.

x bedeutet bevorzugt die Zahl 1, 2 oder 3 und besonders bevorzugt die Zahl 1 oder 2. y steht vorzugsweise für die Zahl 0 oder 1 und insbesondere für die Zahl 0. Bei den Substituenten L₁ und L₂ handelt es sich unabhängig voneinander je bevorzugt um Sulfo, C₁-C₄-Alkyl oder C₁-C₁₂-Alkoxy und besonders bevorzugt um Sulfo oder C₁-C₄-Alkoxy.

Beispiele für geeignete Oxalanilidreste U sind der Rest von 4,4'-Di-octyloxy-oxanilid, 2,2'-Diethoxy-oxanilid, 2,2'-Di-octyloxy-5,5'-di-tert-butyl-oxanilid, 2,2'-Didodecyloxy-5,5'di-tert-butyl-oxanilid, 2-Ethoxy-2'-ethyl-oxanilid, 2-Methoxy-5-sulfooxanilid, 2-Ethoxy-5-sulfooxanilid, 2,5-Dimethoxyoxanilid, 2-Ethoxy-5-tertbutyl-2'-ethyloxanilid gegebenenfalls im Gemisch mit dem Rest von 2-Ethoxy-2'-ethyl-5,4'-di-tert-butyl-oxanilid, oder Gemische der Reste von o- und p-Methoxy- sowie von o-und p-Ethoxy-di-substituierten Oxaniliden.

U steht bevorzugt für einen Rest der Formel 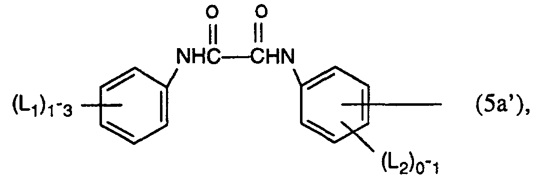 worin (L₁)₁₋₃ für 1 bis 3 gleiche oder verschiedene Substituenten L₁ aus der Gruppe Sulfo, C₁-C₄-Alkyl und C₁-C₁₂-Alkoxy steht und (L₂)₀₋₁ 0 bis 1 Substituenten L₂ aus der Gruppe Sulfo, C₁-C₄-Alkyl und C₁-C₁₂-Alkoxy bedeutet.

U steht besonders bevorzugt für einen Rest der Formel 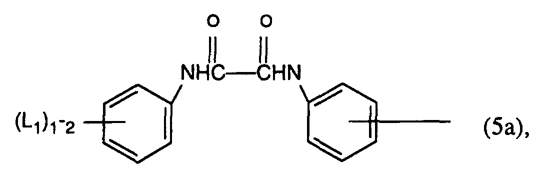 worin (L₁)₁₋₂ für 1 oder 2 Reste L₁ aus der Gruppe Sulfo, C₁-C₄-Alkyl und C₁-C₁₂-Alkoxy und insbesondere Sulfo und C₁-C₄-Alkoxy steht.

W steht bevorzugt für den Rest -NH-.

Steht X₁ in Formel (1) für gegebenenfalls substituiertes Amino, so ist darunter -NH₂, unsubstituiertes oder im Alkylteil durch Hydroxy, Carboxy, Sulfo, Sulfato oder C₁-C₄-Alkoxy substituiertes N-Mono- oder N,N-Di-C₁-C₄-Alkylamino, Cyclohexylamino oder unsubstituiertes oder im Phenylteil durch C₁-C₄-Alkyl, C₁-C₄-Alkoxy, Carboxy, Sulfo, Halogen oder durch einen Rest der Formel

-SO₂-Y (6a),

-CONH-(CH₂)ₚ-SO₂-Y (6b),

worin Y die zuvor angegebene Bedeutung hat und p eine ganze Zahl 1 bis 6 bedeutet, substituiertes Phenylamino oder N-C₁-C₄-Alkyl-N-phenylamino oder einfach oder mehrfach durch Sulfo substituiertes 1- oder 2-Naphthylamino zu verstehen.

In Formel (6b) bedeutet p bevorzugt die Zahl 2, 3 oder 4 und besonders bevorzugt die Zahl 2 oder 3.

X₁ steht als gegebenenfalls substituiertes Amino bevorzugt für Amino, Methylamino, Ethylamino, Carboxymethylamino, β-Hydroxyethylamino, β-Sulfoethylamino, N,N-Di-β-hydroxyethylamino, Cyclohexylamino, o-, m- oder p-Methylphenylamino, o-, m- oder p-Methoxyphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino, Mono-, Di- oder Trisulfonaphthylamino, oder für Phenylamino, welches durch einen Rest der Formel (6a) oder (6b) substituiert ist. Besonders bevorzugt sind für X₁ als gegebenenfalls substituiertem Amino die Bedeutungen Amino, β-Sulfoethylamino, β-Hydroxyethylamino, N,N-Di-β-hydroxyethylamino und o-, m- oder p-Sulfophenylamino.

Bevorzugte Bedeutungen von X₁ sind Chlor, Fluor, Hydroxy, Amino, unsubstituiertes oder im Alkylteil durch Hydroxy, Sulfo oder Sulfato substituiertes N-Mono- oder N,N-Di-C₁-C₄-Alkylamino oder unsubstituiertes oder im Phenylteil durch Methyl, Ethyl, Methoxy, Ethoxy, Carboxy, Sulfo, Chlor oder durch einen Rest der Formel (6a) oder (6b) substituiertes Phenylamino.

Besonders bevorzugt steht X₁ für Chlor oder Fluor.

Steht die Variable =E- für eine Gruppe =C(T₁)-, bedeutet T₁ bevorzugt Halogen und insbesondere Fluor oder Chlor. Die Variable =E- bedeutet bevorzugt die Gruppe =N-.

Bevorzugt ist Z eine Gruppe der Formel -Cl, -OSO₃H, -SSO₃H, -OCO-CH₃, -OCO-C₆H₅ oder -OPO₃H₂, insbesondere -Cl oder -OSO₃H, vorzugsweise -OSO₃H.

Y bedeutet bevorzugt Vinyl, β-Chlorethyl, β-Sulfatoethyl, β-Thiosulfatoethyl, β-Acetoxyethyl, β-Phenoxyethyl oder β-Phosphatoethyl und besonders bevorzugt β-Sulfatoethyl oder Vinyl.

Bei alk und alk' handelt es sich unabhängig voneinander z.B. um einen Methylen-, Ethylen-,1,3-Propylen-, 1,4-Butylen-, 1,5-Pentylen- oder 1,6-Hexylenrest oder deren verzweigte Isomere.

Bevorzugt stehen alk und alk' unabhängig voneinander je für einen C₁-C₄-Alkylenrest und insbesondere bevorzugt für einen Ethylenrest oder 1,2- oder 1,3-Propylenrest.
arylen ist vorzugsweise ein unsubstituierter oder z.B. durch Sulfo, Methyl, Methoxy oder Carboxy substituierter 1,3- oder 1,4-Phenylenrest oder ein unsubstituierter oder durch Sulfo substituierter Naphthylenrest und besonders bevorzugt ein unsubstituierter 1,3- oder 1,4-Phenylenrest.

R₁ ist vorzugsweise Wasserstoff oder C₁-C₄-Alkyl, wie Methyl, Ethyl, Propyl, Isopropyl, Butyl, Isobutyl, sek.-Butyl oder tert.-Butyl. Besonders bevorzugt ist R₁ Wasserstoff.

R₃ bedeutet bevorzugt Wasserstoff, Methyl oder Ethyl, insbesondere Wasserstoff.

R₄ bedeutet bevorzugt Wasserstoff.

W₂ steht vorzugsweise für -NH- oder -O- und insbesondere bevorzugt für -O-.

W₃ bedeutet bevorzugt eine Gruppe der Formel -CONH- oder -NHCO-, insbesondere eine Gruppe der Formel -CONH-.

u bedeutet bevorzugt die Zahl 0.

Bevorzugt steht T für einen Rest der zuvor angegebenen Formeln (2a) bis (2e), worin W₃ eine Gruppe der Formel -CONH- oder -NHCO-, R₁, R₃ und R₄ je Wasserstoff, W₂ der Rest -O- oder -NH- , alk und alk' unabhängig voneinander je Ethylen oder Propylen, arylen unsubstituiertes oder durch Methyl, Methoxy, Carboxy oder Sulfo substituiertes Phenylen oder unsubstituiertes oder durch Sulfo substituiertes Naphthylen, Y Vinyl oder β-Sulfatoethyl und u die Zahl 0 bedeuten.

Besonders bevorzugte faserreaktive Reste T entsprechen der Formel 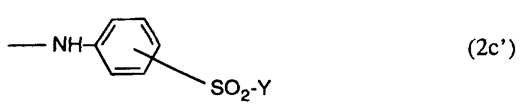 oder 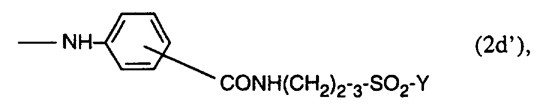 worin Y Vinyl oder β-Sulfatoethyl ist.

Eine bevorzugte Ausführungsform der vorliegenden Erfindung betrifft Verbindungen der Formel 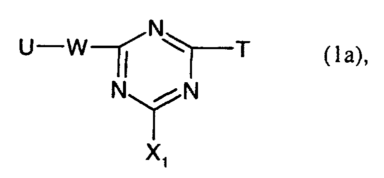 worin W für die Gruppe -NH- steht, X₁ Chlor oder Fluor bedeutet, U einen Rest der Formel 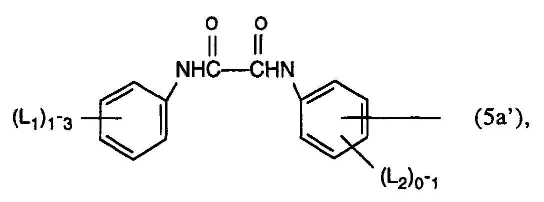 worin (L₁)₁₋₃ für 1 bis 3 gleiche oder verschiedene Substituenten L₁ aus der Gruppe Sulfo, Hydroxy, C₁-C₄-Alkyl und C₁-C₁₂-Alkoxy steht und (L₂)₀₋₁ 0 bis 1 Substituenten L₂ aus der Gruppe Sulfo, C₁-C₄-Alkyl und C₁-C₁₂-Alkoxy bedeutet;
und T für einen Rest der zuvor angegebenen Formel (2a) bis (2e) steht, worin W₃ eine Gruppe der Formel -CONH- oder -NHCO-, R₁, R₃ und R₄ je Wasserstoff, W₂ der Rest -Ooder -NH-, alk und alk' unabhängig voneinander je Ethylen oder Propylen, arylen unsubstituiertes oder durch Methyl, Methoxy, Carboxy oder Sulfo substituiertes Phenylen oder unsubstituiertes oder durch Sulfo substituiertes Naphthylen, Y Vinyl oder β-Sulfatoethyl und u die Zahl 0 bedeuten.

Eine besonders bevorzugte Ausführungsform der vorliegenden Erfindung betrifft Verbindungen der zuvor angegebenen Formel (1a), worin U ein Rest der zuvor angegebenen Formel (5a) ist.

Von besonderem Interesse sind Verbindungen der Formel 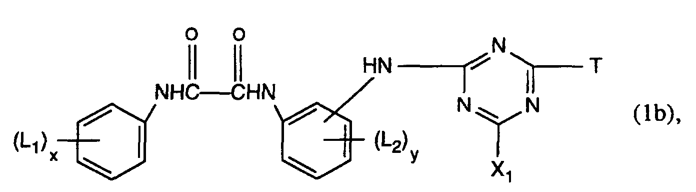 worin für (L₁)ₓ, (L₂)_{y}, T und X₁ jeweils die zuvor angegebenen Bedeutungen und Bevorzugungen gelten.

In den Formeln (1) bis (6b) bedeuten C₁-C₁₂-Alkyl generell z.B. Methyl, Ethyl, n- oder iso-Propyl, n-, iso-, sec- oder tert-Butyl oder geradkettiges oder verzweigtes Pentyl, Hexyl, Heptyl, Octyl, Nonyl, Decyl, Undecyl oder Dodecyl. Unter C₁-C₁₈-Alkoxy ist generell z.B. Methoxy, Ethoxy, n- oder iso-Propoxy, n-, iso-, sec- oder tert-Butoxy oder geradkettiges oder verzweigtes Pentoxy, Hexyloxy, Heptyloxy, Octyloxy, Nonyloxy, Decyloxy, Undecyloxy, Dodecyloxy, Tridecyloxy, Tetradecyloxy, Pentadecyloxyl, Hexadecyloxy, Heptadecyloxy oder Oktadecyloxy zu verstehen. Halogen steht generell z.B. für Fluor, Chlor oder Brom. C₁-C₄-Alkoxycarbonyl bedeutet generell z.B. Methoxycarbonyl, Ethoxycarbonyl, n- oder iso-Propoxycarbonyl oder n-, iso-, sec- oder tert-Butoxycarbonyl. Beispiele für C₁-C₄-Alkylthio sind Methyl- oder Ethylthio.

Die Verbindungen der Formel (1) müssen mindestens eine Sulfo- oder Sulfatogruppe aufweisen, wobei diese jeweils in Form der freien Säure oder vorzugsweise in Salzform, z.B. als Na-, Li-, K- oder Ammoniumsalz, vorliegen kann.

Die Verbindungen der Formel (1) sind faserreaktiv. Unter faserreaktiven Resten sind solche zu verstehen, die mit den Hydroxygruppen der Cellulose, den Amino-, Carboxy-, Hydroxy- und Thiolgruppen bei Wolle und Seide, oder mit den Amino- und eventuell Carboxygruppen von synthetischen Polyamiden unter Bildung kovalenter chemischer Bindungen zu reagieren vermögen.

Die Verbindungen der Formel (1) können hergestellt werden, z.B. indem man eine Verbindung der Formel

U - W - H (7),

eine Verbindung der Formel 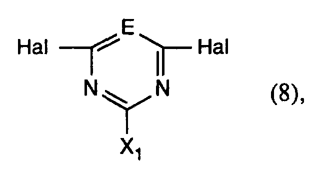 und eine Verbindung der Formel

T*- H (9),

worin U, W, E und X₁ jeweils die zuvor angegebene Bedeutung haben, Hal Halogen, vorzugsweise Fluor oder Chlor, bedeutet und T* die zuvor für T angegebene Bedeutung hat, miteinander umsetzt, wobei die Reihenfolge der Teilreaktionen unter Berücksichtigung der miteinander umzusetzenden Ausgangsverbindungen frei gewählt werden kann.

Eine bevorzugte Variante des Verfahrens besteht darin, dass man in etwa 1 Moläquivalent einer Verbindung der zuvor angegebenen Formel (7) mit in etwa 1 Moläquivalent einer Verbindung der Formel 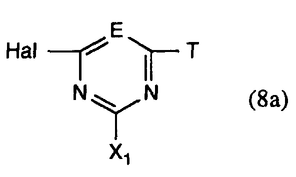 umsetzt, worin Hal, E, X₁ und T jeweils die zuvor angegebene Bedeutung haben.

Die bei der Kondensation der Verbindungen der Formel (7), (8) und (9) anzuwendenden Bedingungen sind aus dem Bereich der Reaktivfarbstoffchemie hinlänglich bekannt. Üblicherweise verlaufen diese Reaktion in einem wässrigen oder wässrig-organischen Medium in Gegenwart von säurebindenden Mitteln, z.B. Natriumcarbonat oder Natriumhydroxid.

Die Verbindungen der Formel (8), (8a) und (9) sind bekannt oder können nach an sich bekannten Methoden erhalten werden.

Die Verbindungen der Formel 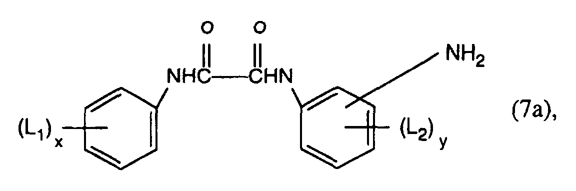 worin für (L₁)ₓ und (L₂)_{y} jeweils die zuvor angegebenen Bedeutungen und Bevorzugungen gelten, sind neu und stellen einen weiteren Gegenstand der Erfindung dar. Sie können hergestellt werden, z.B. indem man ein Nitrooxanilsäurederivat der Formel 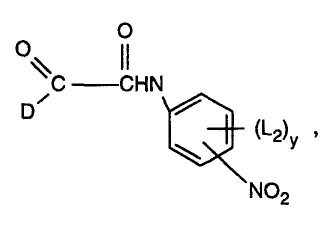 worin D z.B. Halogen oder C₁-C₄-Alkoxy ist und (L₂)_{y} die zuvor angegebene Bedeutung hat, mit einem Anilinderivat der Formel 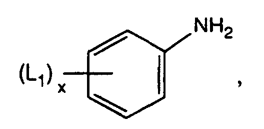 worin (L₁)ₓ die zuvor angegebene Bedeutung hat, umsetzt und die erhaltene Nitrooxanilidverbindung anschliessend katalytisch zur entsprechenden Aminoverbindung hydriert.

Die neuen UV-Absorber der Formel (1) eignen sich zum photochemischen Stabilisieren von ungefärbten und gefärbten oder bedruckten Fasermaterialien z.B. aus Seide, Leder, Wolle, Polyamid oder Polyurethanen, und insbesondere von cellulosehaltigen Fasermaterialien aller Art. Solche Fasermaterialien sind beispielsweise die natürliche Cellulosefaser, wie Baumwolle, Leinen, Jute und Hanf, sowie Zellstoff und regerierte Cellulose. Bevorzugt sind textile Fasermaterialien aus Baumwolle. Die Verbindungen der Formel (1) eignen sich auch zum photochemischen Stabilisieren von hydroxylgruppenhaltigen Fasern, die in Mischgeweben enthalten sind, z.B. von Gemischen aus Baumwolle mit Polyesterfasern oder Polyamidfasern. Ein weiteres bevorzugtes Anwendungsgebiet betrifft die Sperrung bzw. Verminderung der durch die genannten Textilmaterialien fallenden UV-Strahlung (UV-Cutting) und den erhöhten Sonnenschutz, den mit einer erfindungsgemässen Verbindung ausgerüstete Textilmaterialien der menschlichen Haut bieten.

Dazu werden eine oder mehrere verschiedene Verbindungen der Formel (1) vorteilhaft in einer Menge von 0,01 bis 5 Gew.-%, vorzugsweise 0,1-3, und insbesondere 0,25 bis 2 Gew.-%, bezogen auf das Gewicht des Fasermaterials, nach einem der üblichen Färbeverfahren für Reaktivfarbstoffe auf das textile Fasermaterial aufgebracht. Handelt es sich bei dem Textilfasermaterial um ein mit einem Reaktivfarbstoff gefärbtes cellulosisches Material, so kann das Applizieren des UV-Absorbers der Formel (1) vor, während oder nach der Färbung, vorzugsweise gleichzeitig mit der Applikation des Farbstoffs erfolgen.

Die erfindungsgemässen Verbindungen der Formel (1) lassen sich auf verschiedene Weise auf das Fasermaterial applizieren und auf der Faser fixieren, insbesondere in Form von wässrigen Lösungen oder Druckpasten. Sie besitzen ein gutes Ziehvermögen und eignen sich sowohl für das Ausziehverfahren als auch für die Foulardfärberei, können bei niedrigen Temperaturen eingesetzt werden und erfordern bei Pad-Steam-Verfahren nur kurze Dämpfzeiten. Die Fixiergrade sind hoch, und die nicht-fixierten Anteile können leicht ausgewaschen werden, wobei die Differenz zwischen Ausziehgrad und Fixiergrad bemerkenswert klein ist. Die Verbindungen der Formel (1) eignen sich auch zum Druck, vor allem auf Baumwolle.

Die mit den erfindungsgemässen Verbindungen der Formel (1) ausgerüsteten textilen Fasermaterialien besitzen einen verbesserten Schutz gegen photochemischen Faserabbau und Vergilbungserscheinungen sowie im Fall von gefärbtem Fasermaterial eine verbesserte (Heiss-)Lichtechtheit. Besonders hervorzuheben ist die stark verbesserte Lichtschutzwirkung des behandelten textilen Fasermaterials und hierbei insbesondere die gute Schutzwirkung gegenüber kurzwelligen UV-B-Strahlen. Dies drückt sich dadurch aus, dass das mit einer erfindungsgemässen Verbindung der Formel (1) ausgerüstete textile Fasermaterial einen gegenüber dem unbehandelten Gewebe stark erhöhten Sonnenschutzfaktor (SF) aufweist.

Der Sonnenschutzfaktor wird definiert als Quotient aus schädlicher UV-Strahlungsdosis ohne Sonnenschutz und schädlicher UV-Strahlungsdosis mit Sonnenschutz. Dementsprechend stellt ein Sonnenschutzfaktor auch einen Massstab für die Durchlässigkeit der unbehandelten und der mit einer erfindungsgemässen Verbindung der Formel (1) behandelten Fasermaterialien für UV-Strahlung dar. Die Bestimmung des Sonnenschutzfaktors von textilen Fasermaterialien wird z.B. in der WO 94/04515 oder in J. Soc. Cosmet. Chem. 40, 127-133 (1989) erläutert und kann analog dazu erfolgen.

Vorteilhaft wirkt sich bei den erfindungsgemässen Verbindungen weiterhin aus, dass sie das textile Fasermaterial bei einer entsprechenden Ausrüstung nicht anschmutzen. Die nachfolgenden Beispiele dienen der Erläuterung der Erfindung. Die Temperaturen sind in Celsiusgraden angegeben, Teile sind Gewichtsteile, die Prozentangaben beziehen sich auf Gewichtsprozente, sofern nichts anderes vermerkt ist.

Beispiel 1: Ein Gemisch aus 20 Teilen 3-Nitrooxanilsäureethylester und 23 Teilen o-Phenetidin wird 4 Stunden lang unter Stickstoffatmosphäre auf 140-150°C erhitzt. Danach wird der Druck auf ca. 40 mbar abgesenkt und das Gemisch weitere 4 Stunden bei ca. 140°C gerührt. Man kühlt ab und versetzt den halbfesten Rückstand mit Wasser und 4 n Salzsäure. Dann wird die Verbindung der Formel 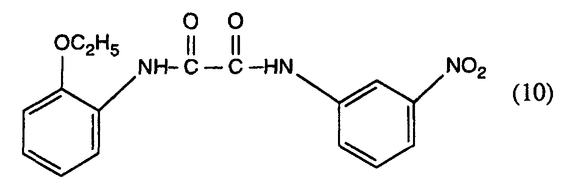 abfiltriert, mit Wasser gewaschen und getrocknet.

Beispiel 1a: Verfährt man wie im Beispiel I beschrieben und verwendet anstelle von 23 Teilen o-Phenetidin 51,9 Teile o-Phenetidin und anstelle von 20 Teilen 3-Nitrooxanilsäureethylester 45 Teile 4-Nitrooxanilsäureethylester, erhält man die Verbindung der Formel

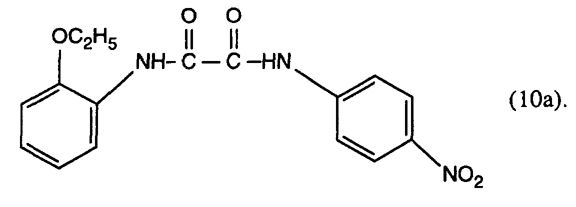

Beispiel 2: Zu 55 Teilen 100%iger Schwefelsäure trägt man innerhalb einer Stunde 9,9 Teile der Verbindung der Formel (10) gemäss Beispiel 1 ein und hält dabei die Temperatur unterhalb von 20°C. Anschliessend lässt man eine Stunde weiterrühren und trägt dann auf Eis aus. Nach Zugabe von Kochsalz wird der erhaltene Feststoff abfiltriert, anschliessend in Kochsalzlösung wieder aufgenommen und durch Behandeln mit Natriumhydroxidlösung das Produkt der Formel 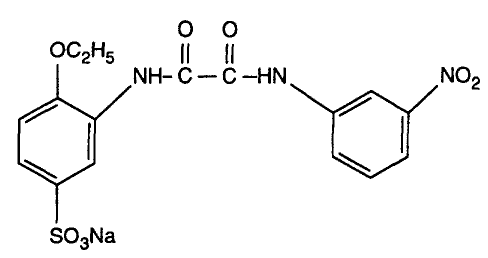 gewonnen.

Beispiel 2a: Verfährt man wie im Beispiel 2 beschrieben und verwendet anstelle der Verbindung der Formel (10) die äquivalente Menge der Verbindung der Formel (10a), erhält man die Verbindung der Formel

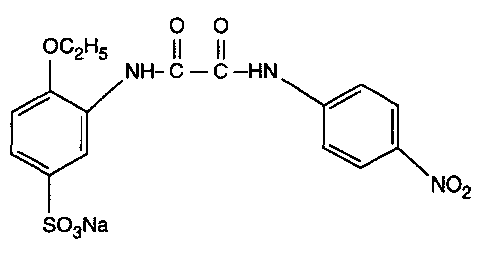

Beispiel 3: Eine Anschlämmung von 12 Teilen der gemäss Beispiel 2 erhaltenen Nitroverbindung in 1000 ml Wasser wird mittels eines Pd/C (5%)-Katalysators katalytisch hydriert und die erhaltene Aminoverbindung der Formel 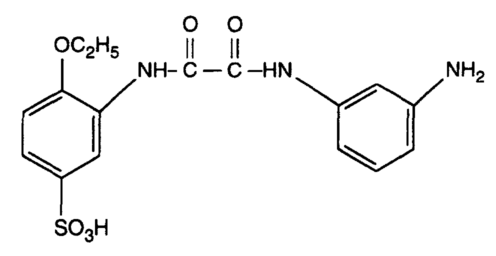 anschliessend in üblicher Weise isoliert und gereinigt.

Beispiel 3a: Analog wie im Beispiel 3 beschrieben lässt sich aus der gemäss Beispiel 2a erhaltenen Nitroverbindung die Verbindung der Formel 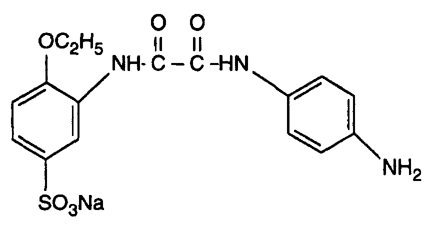 herstellen.

Beispiele 3b-3i: Analog wie in den Beispielen 1 bis 3a beschrieben lassen sich die folgenden Oxanilid-Zwischenprodukte herstellen.

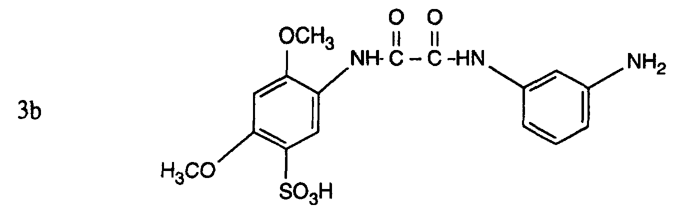

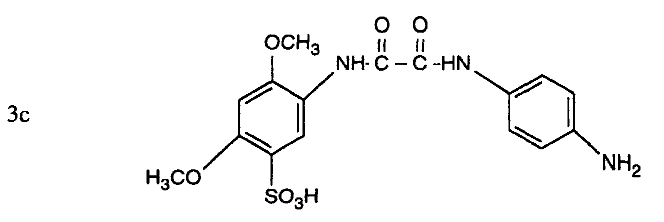

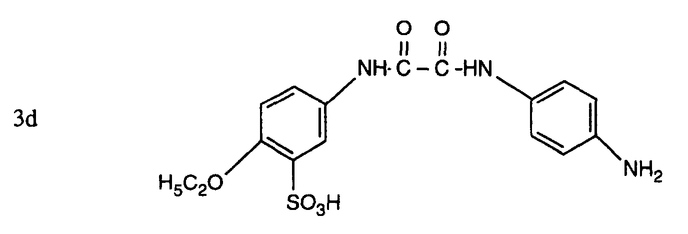

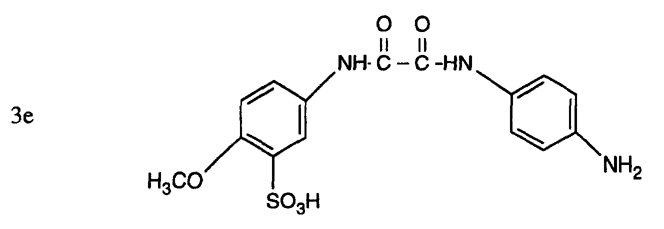

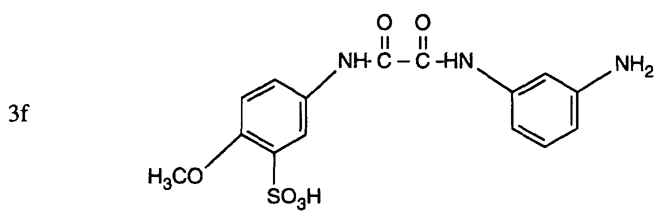

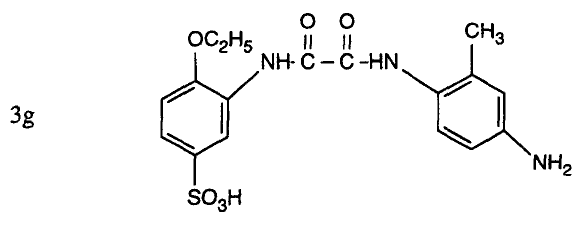

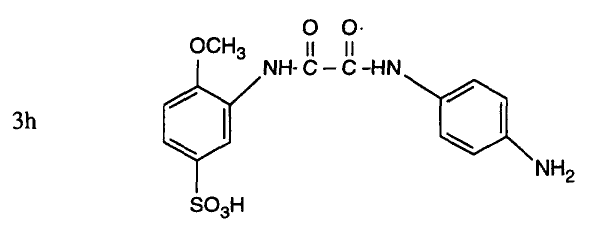

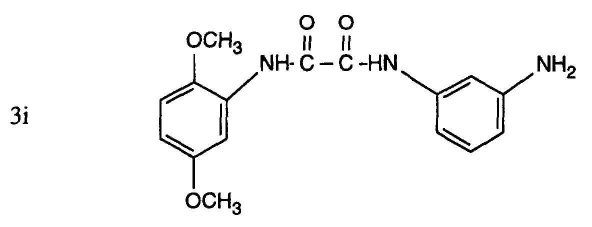

Beispiel 3j: Zu einem Gemisch aus 22,5 Teilen 1,4-Phenylendiamin-2-sulfonsäure (als Na-Salz) und 9,5 Teilen Imidazol in 56 Teilen N,N-Dimethylacetamid wird bei 40°C unter Stickstoffatmosphäre eine Lösung von 28,5 Teilen 2-Ethoxyoxanilsäureethylester in 56 Teilen N,N-Dimethylacetamid zugegeben. Man rührt während ca. 18 Stunden bei 90°C, lässt danach auf 45°C abkühlen und gibt 220 Teile Wasser zum Reaktionsgemisch. Anschliessend wird das Produkt der Formel 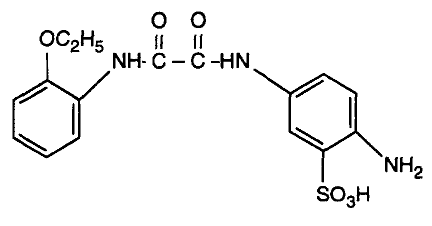 abfiltriert, mit Wasser gewaschen und getrocknet.

Beispiel 3k: Zu 50 Teilen 100%iger Schwefelsäure trägt man innerhalb von 15 Minuten 10 Teile der gemäss Beispiel 3j erhaltenen Verbindung ein und hält dabei die Temperatur bei ca. 20°C. Anschliessend lässt man eine Stunde bei Raumtemperatur weiterrühren und trägt dann auf Eis aus. Nach Zugabe von Kochsalz wird der erhaltene Feststoff abfiltriert, anschliessend in Kochsalzlösung wieder aufgenommen und durch Behandeln mit Natriumhydroxidlösung das Produkt der Formel 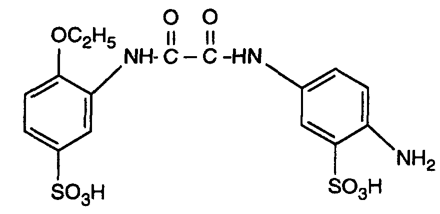 als Natriumsalz isoliert.

Beispiele 3l bis 3r: Analog wie in den Beispielen 3j und 3k beschrieben lassen sich die folgenden Oxanilid-Zwischenprodukte herstellen.

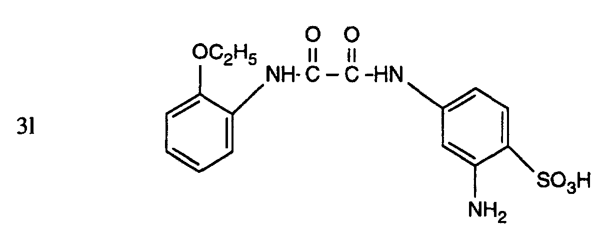

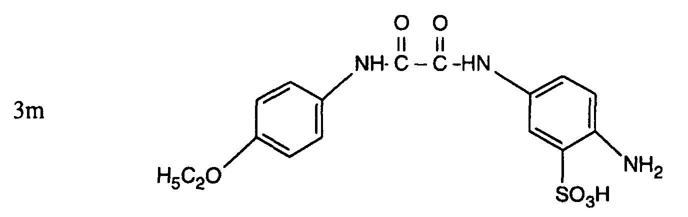

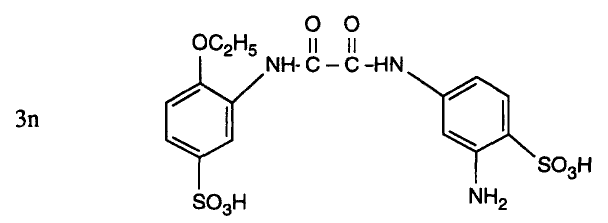

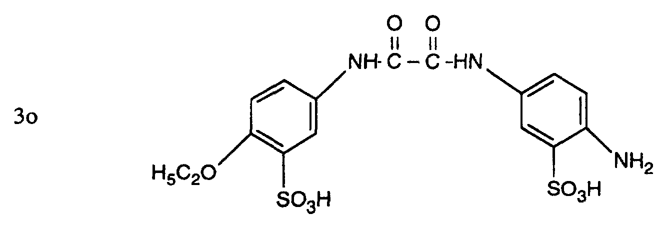

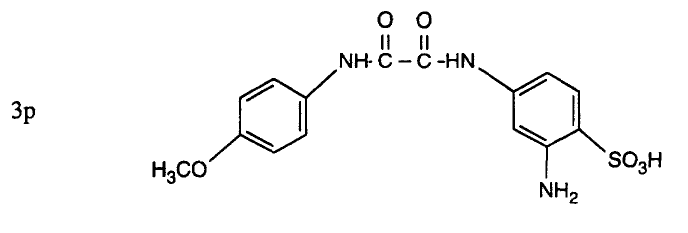

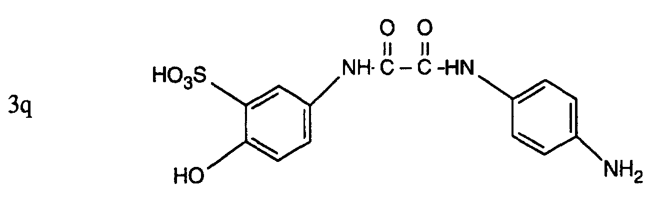

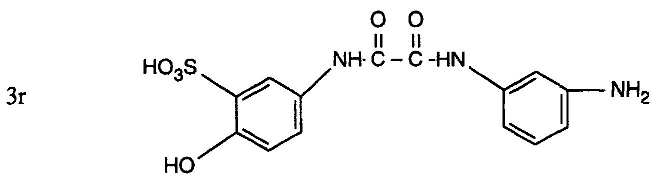

Beispiel 4: Eine eiskalte wässrige Suspension des Kondensationsprodukts aus 1,84 Teilen Cyanurchlorid und 2,81 Teilen 4-β-Sulfatoethylsulfonylanilin (Herstellung gemäss DE-A 3,740,650) wird mit einer wässrigen Anschlämmung von 3,79 Teilen der gemäss Beispiel 3 erhaltenen Aminoverbindung versetzt. Man erhöht die Reaktionstemperatur auf 35°C und lässt bis zur vollständigen Kondensation rühren, wobei der pH-Wert des Gemisches durch Zutropfen von 15%iger Sodalösung konstant bei 7 gehalten wird. Das Produkt wird anschliessend in üblicher weise ausgesalzen, abfiltriert, mit Kochsalzlösung gewaschen und getrocknet. Man erhält die Verbindung der Formel 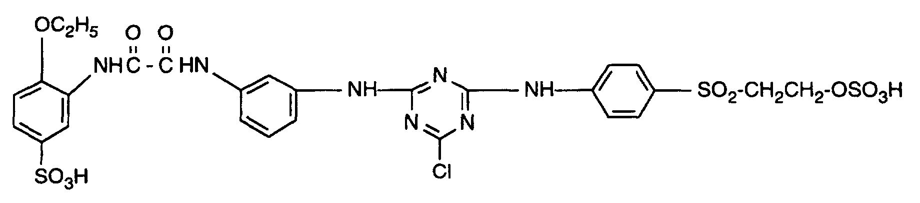 als Pulver. Bringt man die Verbindung mittels eines üblichen Färbeverfahrens für Reaktivfarbstoffe auf ein Baumwollgewebe auf, so weist dieses einen gegenüber der unbehandelten Ware stark erhöhten Sonnenschutzfaktor auf.

Beispiel 4a: Analog wie im Beispiel 4 beschrieben lässt sich aus 7,6 Teilen der gemäss Beispiel 3a erhaltenen Aminoverbindung und 8,6 Teilen des im Beispiel 4 beschriebenen Kondensationsprodukts aus Cyanurchlorid und 4-β-Sulfatoethylsulfonylanilin die Verbindung der Formel 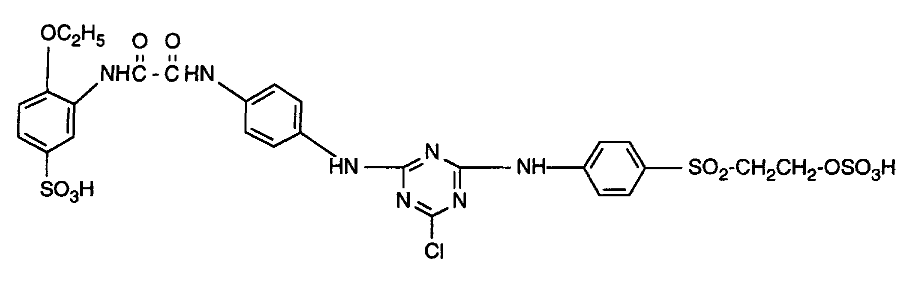 herstellen. Bringt man die Verbindung mittels eines üblichen Färbeverfahrens für Reaktivfarbstoffe auf ein Baumwollgewebe auf, so weist dieses einen gegenüber der unbehandelten Ware stark erhöhten Sonnenschutzfaktor auf.

Beispiele 5-11: Analog wie in den Beispielen 1, 2, 3 und 4 beschrieben lassen sich die Verbindungen der Formel 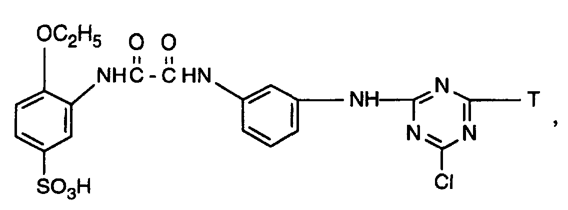 worin T die in der Tabelle angegebene Bedeutung hat, herstellen; diese ergeben auf Baumwolle ausgefärbt einen ähnlich guten Sonnenschutzfaktor wie die Verbindung gemäss Beispiel 4.

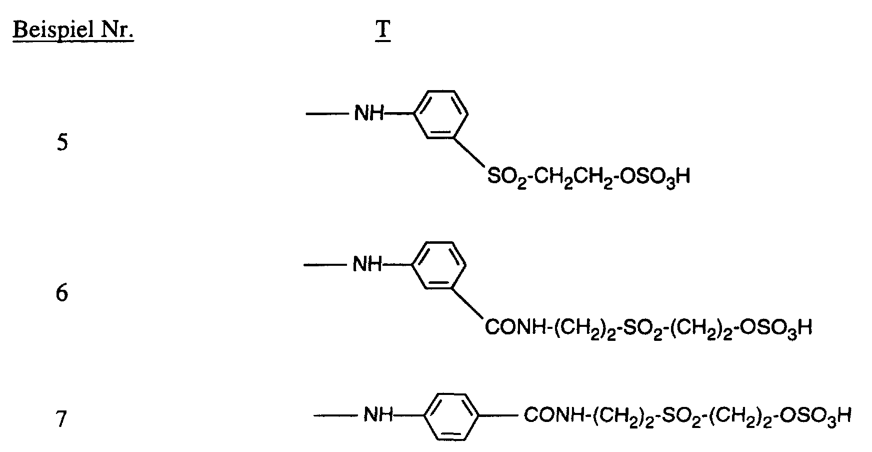

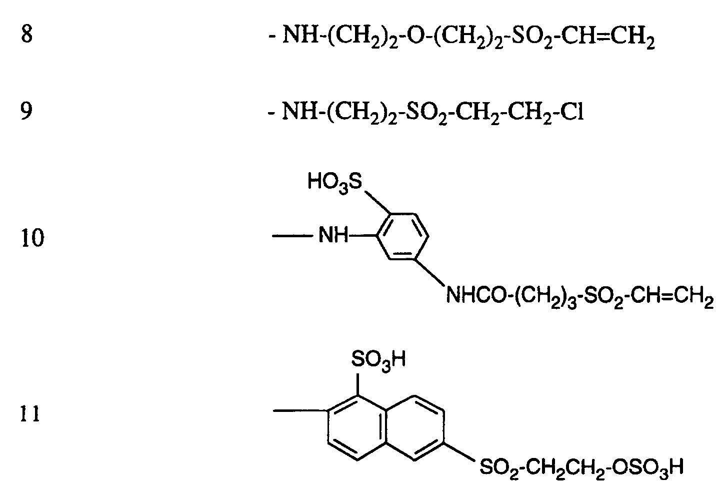

Beispiele 12-28: Analog wie im Beispiel 4 beschrieben lassen sich aus den Zwischenprodukten gemäss den Beispielen 3 bis 3r die folgenden Verbindungen herstellen, welche mittels eines üblichen Färbeverfahrens für Reaktivfarbstoffe auf ein Baumwollgewebe aufgebracht diesem einen gegenüber der unbehandelten Ware stark erhöhten Sonnenschutzfaktor verleihen.

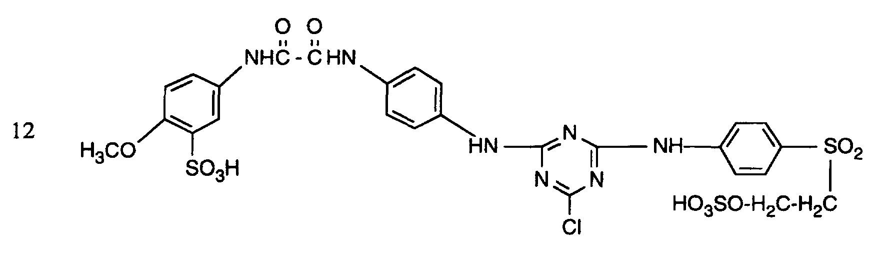

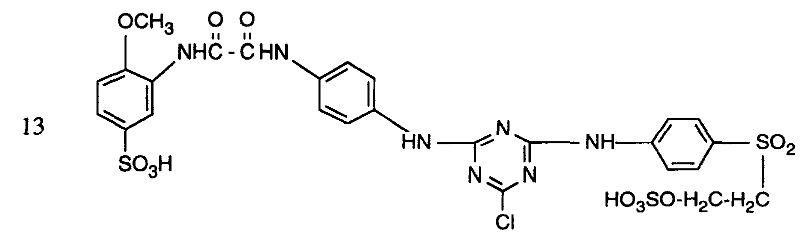

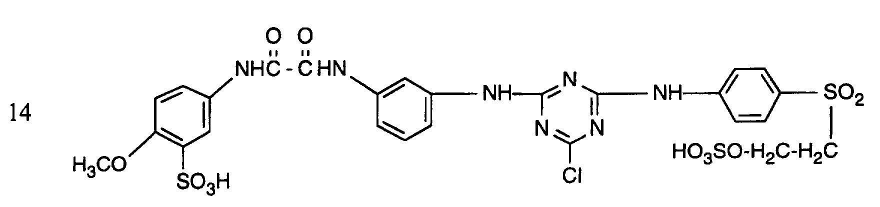

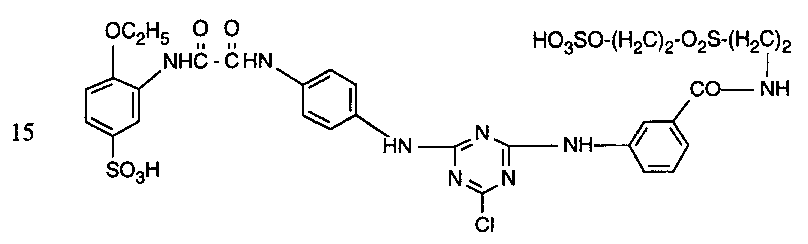

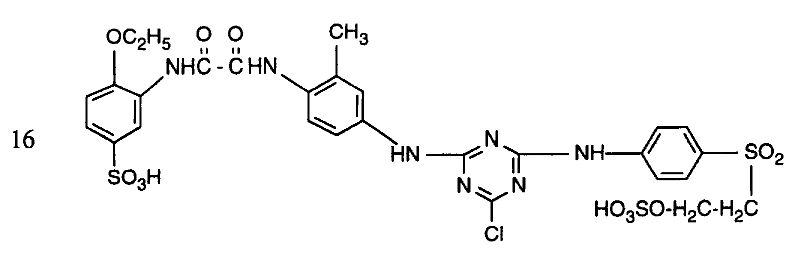

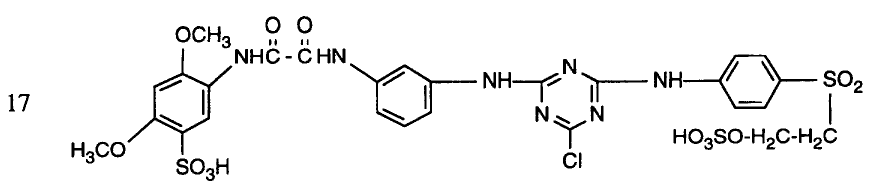

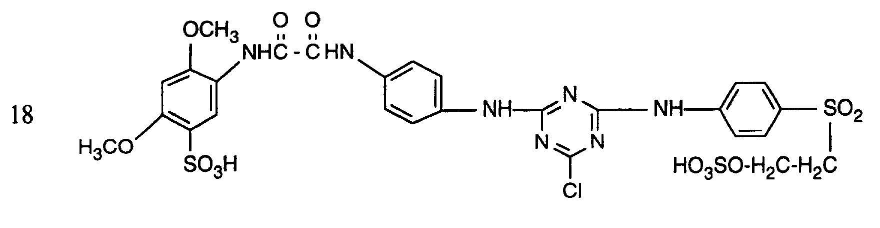

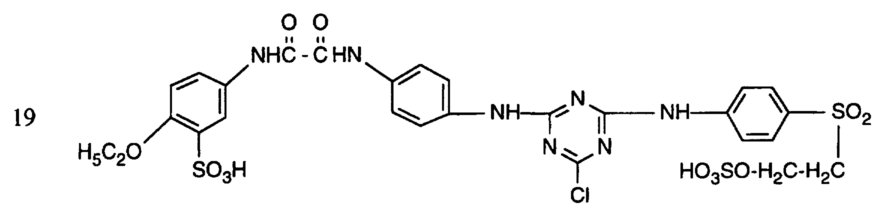

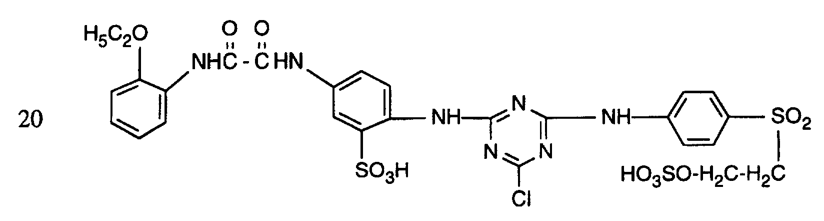

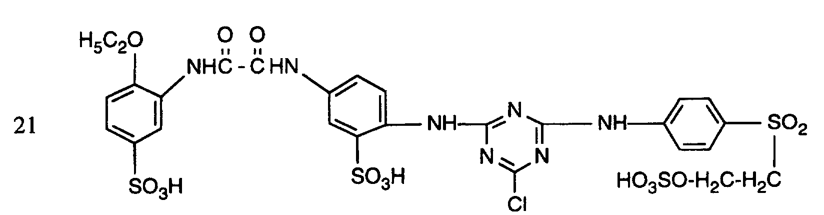

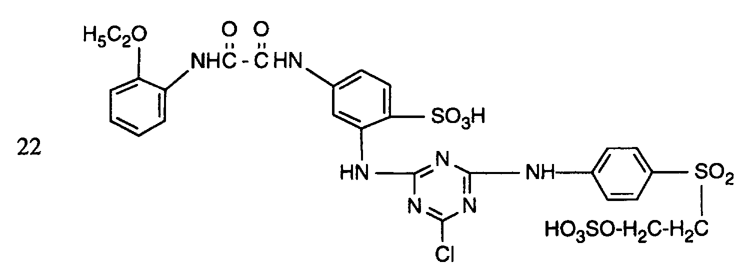

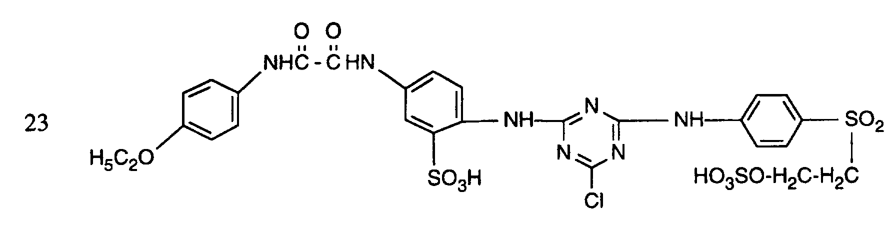

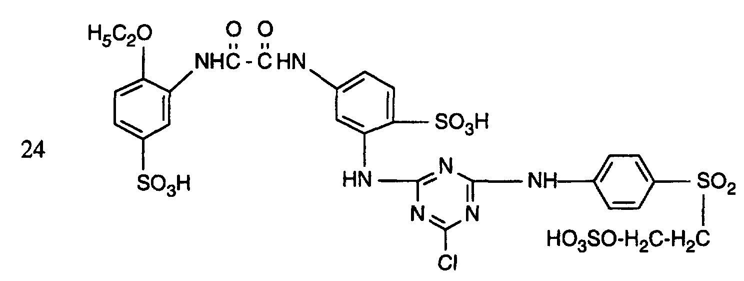

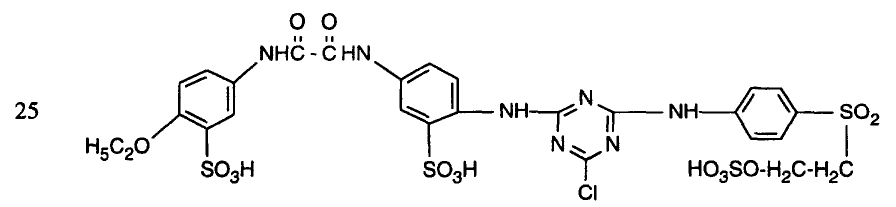

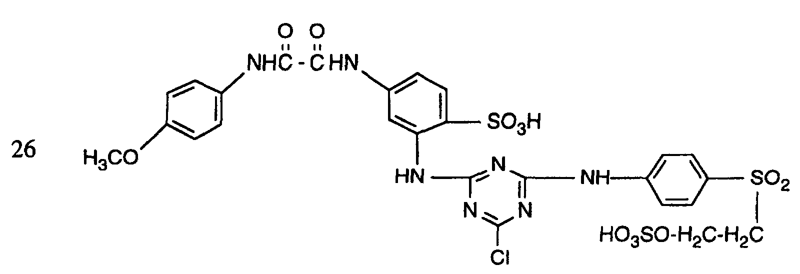

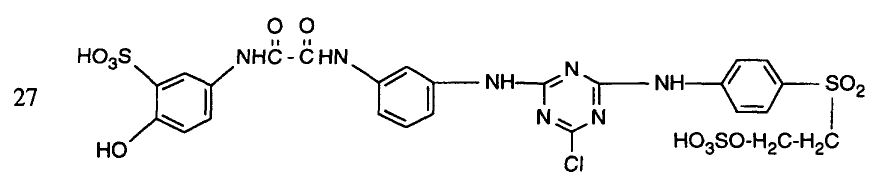

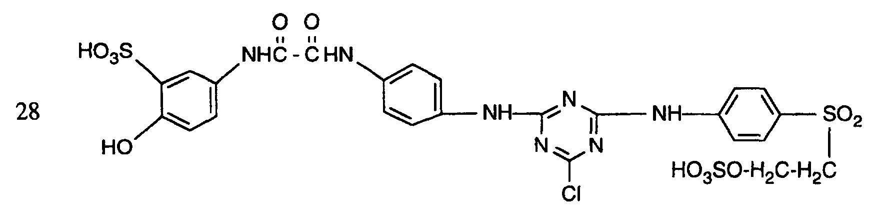

Applikationsbeispiel: In einem Jet-Färbeapparat werden 100 g eines gebleichten Baumwolltrikots 20 Minuten bei 60°C mit einer Flotte enthaltend 1 g der Verbindung gemäss Beispiel 4 und 75 g Natriumsulfat bei einem Flottenverhältnis von 1:15 behandelt. Nach Zugabe von 30 g Natriumcarbonat wird das Baumwolltrikot weitere 60 Minuten bei 60°C behandelt. Dann wird das Fasermaterial der Flotte entnommen, mehrmals mit kaltem, warmen und heissem Wasser gewaschen und getrocknet.
Man erhält ein Baumwolltrikot mit einem ausgezeichneten Sonnenschutzfaktor.

## Patentansprüche

1. Verbindungen der Formel 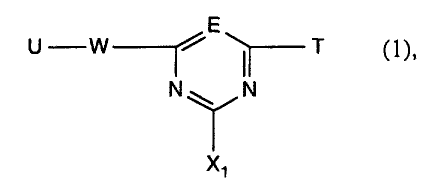 worin
U für den Rest eines Oxalsäurediamids der Formel 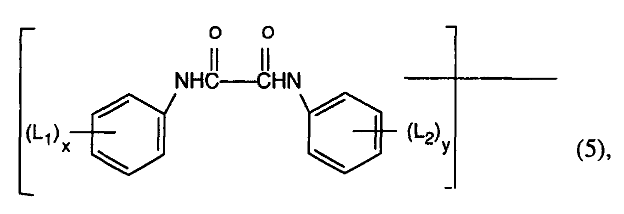 steht, worin (L₁)ₓ für x gleiche oder verschiedene Substituenten L₁ aus der Gruppe Sulfo, unsubstituiertes oder im Alkylteil durch Sulfo substituiertes Alkyl, Alkoxy oder Alkylthio mit je 1 bis 22 Kohlenstoffatomen und unsubstituiertes oder im Phenylring durch Sulfo substituiertes Phenoxy oder Phenylthio steht, (L₂)_{y} y gleiche oder verschiedene Substituenten L₂ aus der Gruppe Sulfo, unsubstituiertes oder im Alkylteil durch Sulfo substituiertes Alkyl, Alkoxy oder Alkylthio mit je 1 bis 22 Kohlenstoffatomen und unsubstituiertes oder im Phenylring durch Sulfo substituiertes Phenoxy oder Phenylthio bedeutet, und x und y unabhängig voneinander je eine ganze Zahl von 0 bis 3 bedeuten, wobei die Summe von (x + y) ≥ 1 ist,
W eine Gruppe -NR₂- bedeutet,
R₂ für Wasserstoff oder unsubstituiertes oder durch Halogen, Hydroxy, Cyano, C₁-C₄-Alkoxy, C₁-C₄-Alkoxycarbonyl, Carboxy, Sulfamoyl, Sulfo oder Sulfato substituiertes C₁-C₄-Alkyl steht,
=E- für eine Gruppe =N- oder =C(T₁)- steht und T₁ Halogen, C₁-C₄-Alkylsulfonyl, Formyl, C₂-C₄-Alkoxycarbonyl oder Cyano ist,
X₁ Halogen, Hydroxy, Sulfo, C₁-C₄-Alkylsulfonyl, Phenylsulfonyl, Amino, unsubstituiertes oder im Alkylteil durch Hydroxy, Carboxy, Sulfo, Sulfato oder C₁-C₄-Alkoxy substituiertes N-Mono- oder N,N-Di-C₁-C₄-Alkylamino, Cyclohexylamino, unsubstituiertes oder im Phenylteil durch C₁-C₄-Alkyl, C₁-C₄-Alkoxy, Carboxy, Sulfo, Halogen oder durch einen Rest der Formel
-SO₂-y (6a),
-CONH-(CH₂)ₚ-SO₂-Y (6b),
worin p eine ganze Zahl 1 bis 6 bedeutet, substituiertes Phenylamino oder N-C₁-C₄-Alkyl-N-phenylamino, einfach oder mehrfach durch Sulfo substituiertes 1- oder 2-Naphthylamino, 3-Carboxypyridin-1-yl oder 3-Carbamoylpyridin-1-yl bedeutet, T ein Reaktivrest der Formel 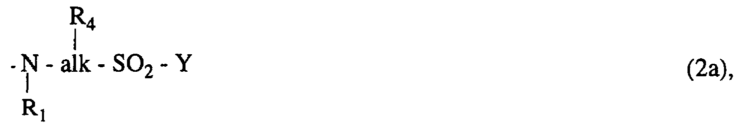 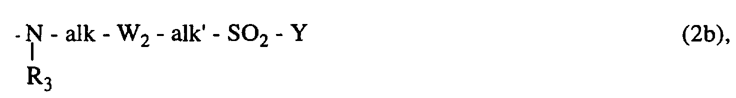 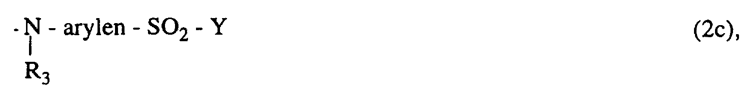 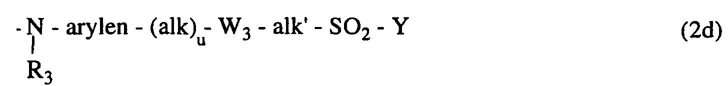 oder 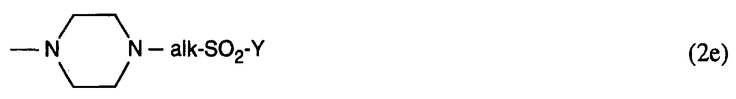 ist,
R₁ Wasserstoff, gegebenenfalls durch Hydroxy, Sulfo, Sulfato, Carboxy oder Cyano substituiertes C₁-C₄-Alkyl oder einen Rest 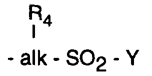 bedeutet,
R₃ für Wasserstoff oder C₁-C₄-Alkyl steht,
R₄ Wasserstoff, Hydroxy, Sulfo, Sulfato, Carboxy, Cyano, Halogen, C₁-C₄-Alkoxycarbonyl, C₁-C₄-Alkanoyloxy, Carbamoyl oder die Gruppe -SO₂-Y ist, alk und alk' unabhängig voneinander C₁-C₆-Alkylen sind,
arylen einen unsubstituierten oder durch Sulfo, Carboxy, C₁-C₄-Alkyl, C₁-C₄-Alkoxy oder Halogen substituierten Phenylen- oder Naphthylenrest bedeutet
Y Vinyl oder ein Rest -CH₂-CH₂-Z und Z eine Gruppe -Cl, -Br, -F, -OSO₃H, -SSO₃H, -OCO-CH₃, -OPO₃H₂, -OCO-CCl₃, -OCO-CHCl₂, -OCO-CH₂Cl, -OSO₂-C₁-C₄-Alkyl, -OSO₂-N(C₁-C₄-Alkyl)₂ oder -OCO-C₆H₅ ist,
W₂ den Rest -O- oder -NR₃- bedeutet,
W₃ für eine Gruppe -SO₂-NR₁-, -CONR₁- oder -NR₁CO- steht,
u die Zahl 0 oder 1 bedeutet, mit der Massgabe, dass die Verbindungen der Formel (1) mindestens eine Sulfo- oder Sulfatogruppe aufweisen.

2. Verbindungen gemäss Anspruch 1, **dadurch gekennzeichnet, dass** =E- für die Gruppe =N- steht bedeuten.

3. Verbindungen gemäss einem der Ansprüche 1 und 2, **dadurch gekennzeichnet, dass** U einen Rest der Formel 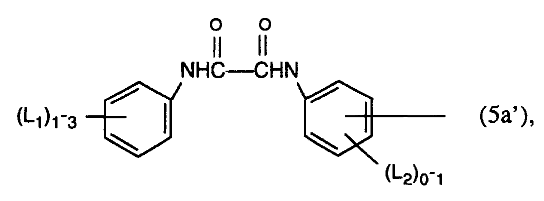 bedeutet, worin (L₁)₁₋₃ für 1 bis 3 gleiche oder verschiedene Substituenten L₁ aus der Gruppe Sulfo, Hydroxy, C₁-C₄-Alkyl und C₁-C₁₂-Alkoxy steht und (L₂)₀₋₁ 0 bis 1 Substituenten L₂ aus der Gruppe Sulfo, C₁-C₄-Alkyl und C₁-C₁₂-Alkoxy bedeutet.

4. Verbindungen gemäss einem der Ansprüche 1 bis 3, **dadurch gekennzeichnet, dass** X₁ für Chlor oder Fluor steht.

5. Verbindungen gemäss einem der Ansprüche 1 bis 4, **dadurch gekennzeichnet, dass** W ein Rest -NR₂- ist und R₂ Wasserstoff oder C₁-C₄-Alkyl bedeutet.

6. Verbindungen gemäss einem der Ansprüche 1 bis 5, **dadurch gekennzeichnet, dass** in den Formeln (2a) bis (2e) W₃ eine Gruppe der Formel -CONH- oder -NHCO-, R₁, R₃ und R₄ je Wasserstoff, W₂ der Rest -O- oder -NH-, alk und alk' unabhängig voneinander je Ethylen oder Propylen, arylen unsubstituiertes oder durch Methyl, Methoxy, Carboxy oder Sulfo substituiertes Phenylen oder unsubstituiertes oder durch Sulfo substituiertes Naphthylen, Y Vinyl oder β-Sulfatoethyl und u die Zahl 0 bedeuten.

7. Verbindungen gemäss Anspruch 1 der Formel 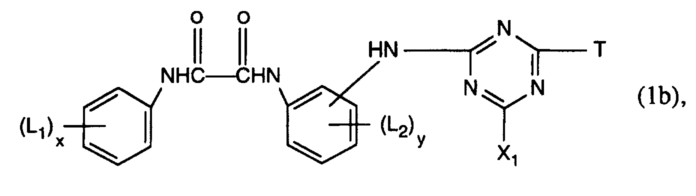 worin (L₁)ₓ für x gleiche oder verschiedene Substituenten L₁ aus der Gruppe Sulfo, Hydroxy, C₁-C₄-Alkyl und C₁-C₁₂-Alkoxy steht, (L₂)_{y} y Substituenten L₂ aus der Gruppe Sulfo, C₁-C₄-Alkyl und C₁-C₁₂-Alkoxy bedeutet, x für die Zahl 1, 2 oder 3 und y für die Zahl 0 oder 1 steht, X₁ Chlor oder Fluor bedeutet und T für einen faserreaktiven Rest der Formel 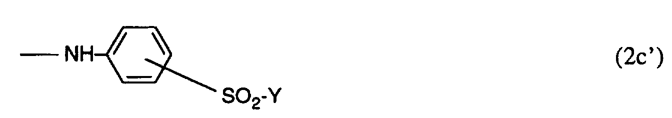 oder 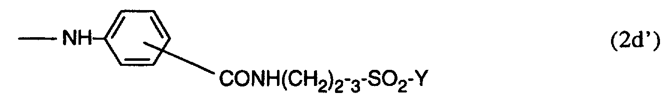 steht, worin Y Vinyl oder β-Sulfatoethyl ist.

8. Verfahren zur Herstellung von Verbindungen der Formel (1) gemäss Anspruch 1, **dadurch gekennzeichnet, dass** man eine Verbindung der Formel
U- W-H (7),
eine Verbindung der Formel 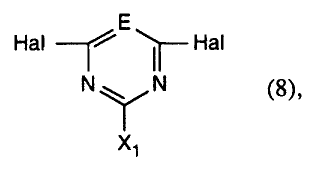 und eine Verbindung der Formel
T* - H (9),
worin U, E, W und X₁ jeweils die im Anspruch 1 angegebene Bedeutung haben, Hal Halogen, vorzugsweise Fluor oder Chlor, bedeutet und T* die im Anspruch 1 für T angegebene Bedeutung hat, in beliebiger Reihenfolge miteinander umsetzt.

9. Verfahren zur Erhöhung des Sonnenschutzfaktors von textilen Fasermaterialien, **dadurch gekennzeichnet, dass** man eine oder mehrere Verbindungen der Formel (1) gemäss Anspruch 1 in einer wässrigen oder wässrig-organischen Lösung auf die Fasermaterialien aufbringt und anschliessend fixiert.

10. Verwendung von Verbindungen der Formel (1) gemäss Anspruch 1 zur Erhöhung des Lichtschutzfaktors von ungefärbten, gefärbten oder bedruckten textilen Fasermaterialien.

11. Verwendung von Verbindungen der Formel (1) gemäss Anspruch 1 zur photochemischen Stabilisierung von ungefärbten, gefärbten oder bedruckten textilen Fasermaterialien.

12. Verbindungen der Formel 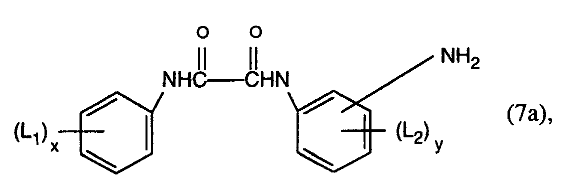 worin (L₁)ₓ und (L₂)_{y} jeweils die im Anspruch 7 angegebene Bedeutung haben.

## Claims

1. A compound of formula 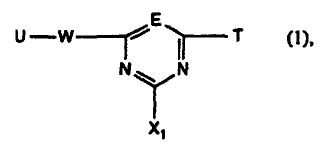 in which
U is the radical of an oxalamide of formula 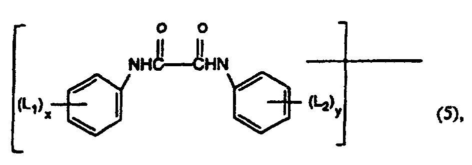 in which (L₁)ₓ is x identical or different substituents L₁ from the group consisting of sulfo, alkyl, alkoxy or alkylthio each having 1 to 22 carbon atoms and being unsubstituted or substituted in the alkyl moiety by sulfo, and phenoxy or phenylthio each unsubstituted or substituted in the phenyl ring by sulfo, (L₂)_{y} is y identical or different substituents L₂ from the group consisting of sulfo, alkyl, alkoxy or alkylthio each having 1 to 22 carbon atoms and being unsubstituted or substituted in the alkyl moiety by sulfo, and phenoxy or phenylthio each unsubstituted or substituted in the phenyl ring by sulfo, and x and y independently of one another are each an integer from 0 to 3, the sum of (x + y) being ≥ 1,
W is a group -NR₂-,
R₂ is hydrogen or unsubstituted or halo-, hydroxyl-, cyano-, C₁-C₄alkoxy-, C₁-C₄-alkoxycarbonyl-, carboxyl-, sulfamoyl-, sulfo- or sulfo-substituted C₁-C₄alkyl,
=E- is a group =N- or =C(T₁)-, and T₁ is halogen, C₁-C₄alkylsulfonyl, formyl, C₂-C₄alkoxycarbonyl or cyano, X₁ is halogen, hydroxy, sulfo, C₁-C₄alkylsulfonyl, phenylsulfonyl, amino, N-mono- or N,N-di-C₁-C₄alkylamino each of which is unsubstituted or subsitituted in the alkyl moiety by hydroxy, carboxy, sulfo, sulfato or C₁-C₄alkoxy, cyclohexylamino, phenylamino or N-C₁-C₄alkyl-N-phenylamino each of which is unsubstituted or substituted in the phenyl moiety by C₁-C₄alkyl, C₁-C₄alkoxy, carboxy, sulfo, halogen or by a radical of formula
-SO₂-Y (6a),
-CONH-(CH₂)ₚ-SO₂-Y (6a),
in which p is an integer from 1 to 6, mono- or polysulfo-substituted 1- or 2-naphthylamino, 3-carboxypyridin-1-yl or 3-carbamoylpyridin-1-yl,
T is a reactive radical of formula 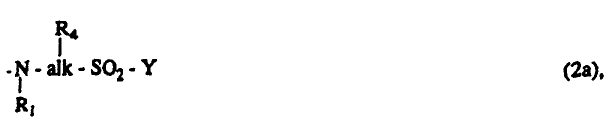 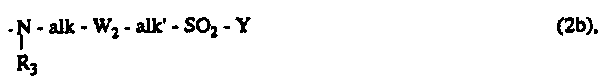 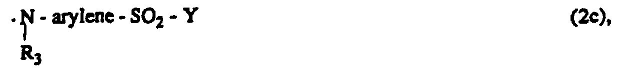 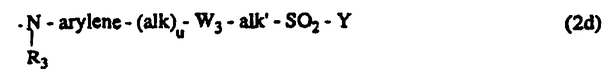 or 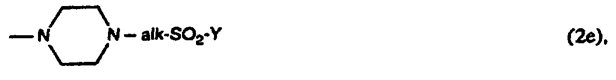 R₁ is hydrogen, C₁-C₄alkyl which is unsubstituted or substituted by hydroxy, sulfo, sulfato, carboxy or cyano, or is a radical 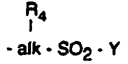 R₃ is hydrogen or C₁-C₄alkyl,
R₄ is hydrogen, hydroxy, sulfo, sulfato, carboxy, cyano, halogen, C₁-C₄alkoxycarbonyl, C₁-C₄alkanoyloxy, carbamoyl or the group -SO₂-Y,
alk and alk' are each independently of the other C₁-C₆alkylene,
arylene is a phenylene or naphthylene radical which is unsubstituted or substituted by sulfo, carboxy, C₁-C₄alkyl, C₁-C₄alkoxy or halogen,
Y is vinyl or a radical -CH₂-CH₂-Z and Z is a -Cl, -Br, -F, -OSO₃H, -SSO₃H, -OCO-CH₃, -OPO₃H₂, -OCO-CCl₃, -OCO-CHCl₂, -OCO-CH₂Cl, -OSO₂-C₁-C₄alkyl, -OSO₂-N(C₁-C₄₋alkyl)₂ or -OCO-C₆H₅,
W₂ is -O- or -NR₃-,
W₃ is a group -SO₂-NR₁-, -CONR₁- or -NR₁CO-, and
u is 0 or 1, with the proviso that the compound of formula (1) has at least one sulfo or sulfato group.

2. A compound according to claim 1, wherein =E- is the group =N-.

3. A compound according to either claim 1 or claim 2, wherein U is a radical of formula 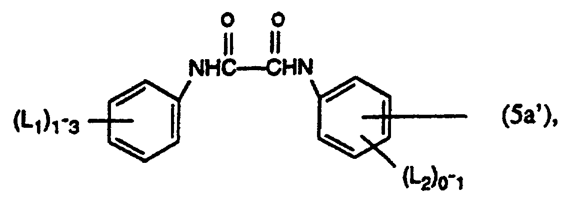 in which (L₁)₁₋₃ is 1 to 3 identical or different substituents L₁ from the group consisting of sulfo, hydroxy, C₁-C₄alkyl and C₁-C₁₂alkoxy and (L₂)₀₋₁ is 0 to 1 substituents L₂ from the group consisting of sulfo, C₁-C₄alkyl and C₁-C₁₂alkoxy.

4. A compound according to any one of claims 1 to 3, wherein X₁ is chloro or fluoro.

5. A compound according to any one of claims to 1 to 4, wherein W is -NR₂- and R₂ is hydrogen or C₁-C₄alkyl.

6. A compound according to any one of claims 1 to 5, wherein in formulae (2a) to (2e) W₃ is a group of formula -CONH- or -NHCO-, R₁, R₃ and R₄ are each hydrogen, W₂ is -O- or -NH-, alk and alk' are each independently of the other ethylene or propylene, arylene is phenylene which is unsubstituted or substituted by methyl, methoxy, carboxy or sulfo, or napthylene which is unsubstituted or substituted by sulfo, Y is vinyl or β-sulfatoethyl, and u is 0.

7. A compound according to claim 1 of formula 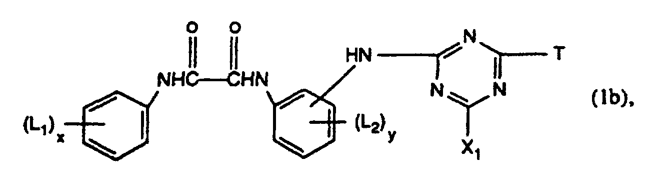 in which (L₁) ₓ denotes x identical or different substituents L₁ from the group consisting of sulfo, hydroxy, C₁-C₄alkyl and C₁-C₁₂alkoxy, (L₂)_{y} denotes y substituents L₂ from the group consisting of sulfo, C₁-C₄alkyl and C₁-C₁₂alkoxy, x is 1, 2 or 3 and y is 0 or 1, X₁ is chloro or fluoro, and T is a fibre-reactive radical of formula 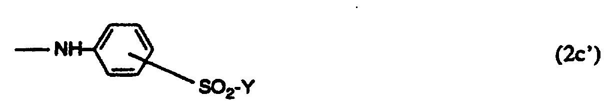 or 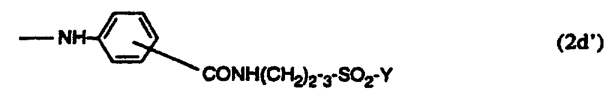 in which Y is vinyl or β-sulfatoethyl.

8. A process for the preparation of a compound of formula (1) according to claim 1, which comprises reacting a compound of formula
U - W - H (7)
a compound of formula 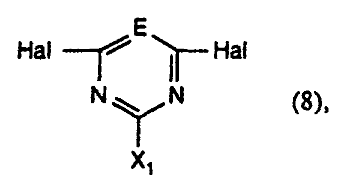 and a compound of formula
T*- H (9),
in which U, E, W and X₁ are each as defined in claim 1, Hal is halogen, preferably fluoro or chloro, and T* has the meaning given in claim 1 for T, with one another, in any order.

9. A process for enhancing the sun protection factor of textile fibre materials, which comprises applying one or more than one compound of formula (1) according to claim 1, in an aqueous or aqueous-organic solution, to said materials, and subsequently fixing said compound or compounds thereon.

10. Use of a compound of formula (1) according to claim 1 for enhancing the sun protection factor of undyed, dyed or printed textile fibre materials.

11. Use of a compound of formula (1) according to claim 1 for photochemically stabilising undyed, dyed or printed textile fibre materials.

12. A compound of formula 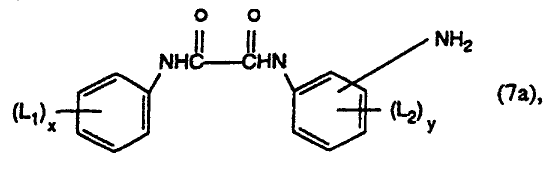 in which (L₁)ₓ and (L₂)_{y} are each as defined in claim 7.

## Revendications

1. Composés de formule 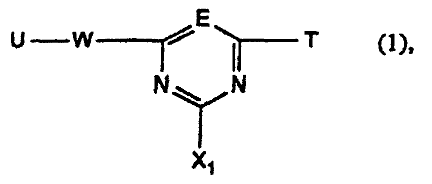 dans laquelle
U représente le reste d'un diamide d'acïde oxalique de formule 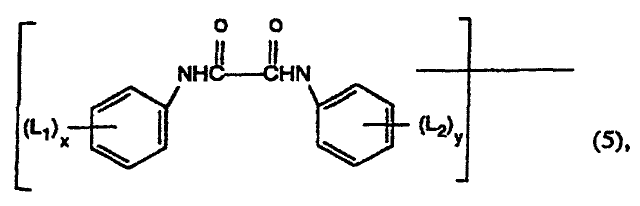 où (L₁)ₓ représente x substituants L₁ identiques ou différents pris dans le groupe comprenant des groupes sulfo, alkyle, alkoxy ou alkylthio présentant chacun 1 à 22 atomes de carbone, non substitué ou substitué dans le fragment alkyle par un substituant sulfo et un groupe phénoxy ou phénylthio non substitué ou substitué dans le cycle phényle par un substituant sulfo, (L₂)_{y} représente y substituants L₂ identiques ou différents pris dans le groupe comprenant sulfo, alkyle, alkoxy ou alkylthio présentant chacun 1 à 22 atomes de carbone, non substitué ou substitué dans le fragment alkyle par un substituant sulfo et un groupe phénoxy ou phénylthio non substitué ou substitué dans le cycle phényle par un substituant sulfo, et x et y représentent chacun, indépendamment l'un de l'autre, un entier de 0 à 3, la somme de (x + y) ≥1,
W représente un groupe NR₂-,
R₂ représente un atome d'hydrogène ou un groupe alkyle en C₁-C₄ non substitué ou substitué par des substituants halogène, hydroxy, cyano, alkoxy en C₁-C₄, (alkoxy en C₁-C₄)carbonyle, carboxy, sulfamoyle, sulfo ou sulfato,
=E- représente un groupe =N ou =C(T₁)- et T₁ représente un atome d'halogène, des groupes (alkyl en C₁-C₄)-sulfonyle, formyle, (alkoxy en C₁-C₄)carbonyle ou cyano,
X₁ représente un atome d'halogène, des groupes hydroxy, sulfo, (alkyl en C₁-C₄)-sulfonyle, phénylsulfonyle, amino, N-mono- ou N,N-di-(alkyl en C₁-C₄)-amino non substitué ou substitué dans le fragment alkyle par des substituants hydroxy, carboxy, sulfo, sulfato ou alkoxy en C₁-C₄, cyclohexylamino, phénylamino ou N-(alkyl en C₁-C₄)-N-phénylamino non substitué ou substitué dans le fragment phényle par des substituants alkyle en C₁-C₄, alkoxy en C₁-C₄, carboxy, sulfo, halogène ou un reste de formule
**-SO**_{**2**}**-Y (6a)**
**-CONH-(CH**_{**2**}**)**_{**p**}**-SO**_{**2**}**-Y (6b)**
où p va de 1 à 6, 1- ou 2-naphtylamino une ou plusieurs fois substitué par un substituant sulfo, 3-carboxypyridin-1-yle ou 3-carbamoylpyridin-1-yle,
T représente un reste réactif 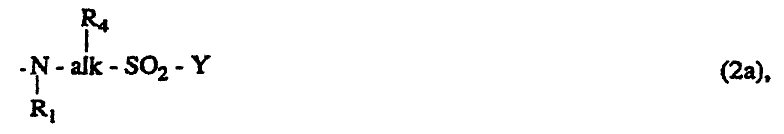 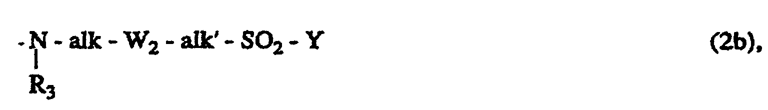 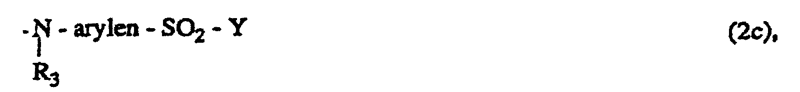 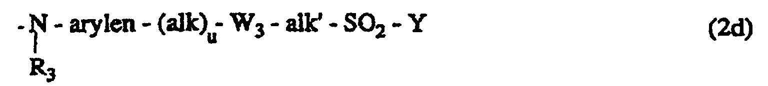 ou 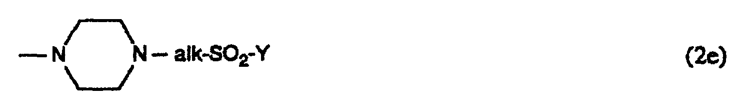 R₁ représente un atome d'hydrogène, un groupe alkyle en C₁-C₄ non substitué ou substitué par des substituants hydroxy, sulfo, sulfato, carboxy ou cyano ou un reste 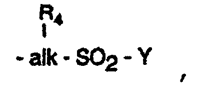 R₃ représente un atome d'hydrogène ou un groupe alkyle en C₁-C₄,
R₄ représente des groupes hydrogène, hydroxy, sulfo, sulfato, carboxy, cyano, halogène (alkoxy en C₁-C₄)-carbonyle, (alcanoyl en C₁-C₄)oxy, carbamoyle ou le groupe -SO₂-Y,
alk et alk' représentent, indépendamment l'un de l'autre, un groupe alkylène en C₁-C₆,
arylen représente un reste phénylène ou naphtylène non substitué ou substitué par des substituants sulfo, carboxy, alkyle en C₁-C₄, alkoxy en C₁-C₄ ou halogène,
Y représente un groupe vinyle ou un reste -CH₂-CH₂-Z et Z représente un groupe -Cl, -Br, -F, -OSO₃H, -SSO₃H, -OCO-CH₃, -OPO₃H₂, -OCO-CCl₃, -OCO-CHCl₂, -OCO-CH₂Cl, -OSO₂-(alkyle en C₁-C₄), -OSO₂-N(alkyle en C₁-C₄)₂ ou -OCOC₆H₅,
W₂ représente le reste -O- ou -NR₃-,
W₃ représente un groupe -SO₂-NR₁-, -CONR₁ ou -NR₁CO-,
u vaut 0 ou 1, à condition que les composés de formule (1) présentent au moins un groupe sulfo ou sulfato.

2. Composés selon la revendication 1, **caractérisés en ce que** =E- représente le groupe =N-.

3. Composés selon l'une des revendications 1 et 2, **caractérisés en ce que** U représente un reste de formule 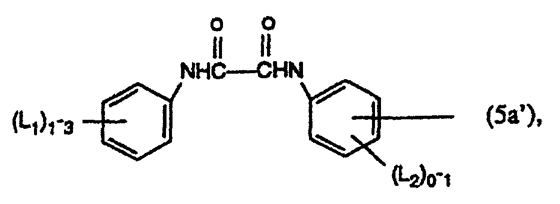 où (L₁)₁₋₃ représente 1 à 3 substituants L₁ identiques ou différents pris dans le groupe comprenant sulfo, alkyle en C₁-C₄ et alkoxy en C₁-C₁₂ et (L₂)₀₋₁ représente 0 à 1 substituant L₂ pris dans le groupe comprenant sulfo, alkyle en C₁-C₄ et alkoxy en C₁-C₁₂.

4. Composés selon l'une des revendications 1 à 3, **caractérisés en ce que** X₁ représente un groupe chloro ou fluoro.

5. Composés selon l'une des revendications 1 à 4, **caractérisés en ce que** W représente un reste -NR₂- et R₂ représente un atome d'hydrogène ou un groupe alkyle en C₁-C₄.

6. Composés selon l'une des revendications 1 à 4, **caractérisés en ce que** dans les formules (2a) à (2e) W₃ représente un groupe de formule -CONH- ou -NHCO-, R₁, R₃ et R₄ représentent chacun un atome d'hydrogène, W₂ représente le reste -O- ou -NH-, alk et alk' représentent chacun indépendamment l'un de l'autre éthylène ou propylène, arylen représente un groupe phénylène non substitué ou substitué par des substituants méthyle, méthoxy, carboxy ou sulfo ou naphtylène non substitué ou substitué par un substituant sulfo, Y représente un groupe vinyle ou β-sulfatoéthyle et u vaut 0.

7. Composés selon la revendication 1 de formule 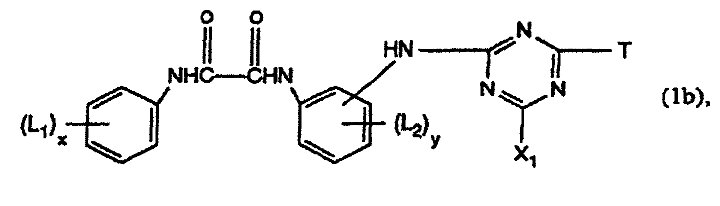 (L₁)ₓ représente x substituants L₁ identiques ou différents pris dans le groupe comprenant sulfo, hydroxy, alkyle en C₁-C₄ et alkoxy en C₁-C₁₂, (L₂)_{y} représente y substituants L₂ identiques ou différents pris dans le groupe comprenant sulfo, alkyle en C₁-C₄ et alkoxy en C₁-C₁₂, x vaut 1, 2 ou 3 et y vaut 0 ou 1, X₁ représente un groupe chloro ou fluoro et T représente un reste réactif sur la fibre de formule 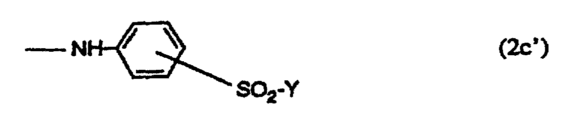 ou 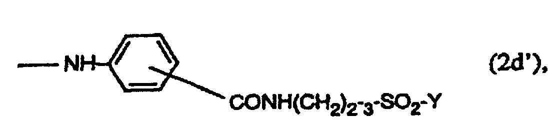 où Y représente groupe vinyle ou β-sulfatoéthyle.

8. Procédé pour la préparation de composés de formule (1) selon la revendication 1, **caractérisé en ce qu'**on fait réagir un composé de formule
**U-W-H (7),**
un composé de formule 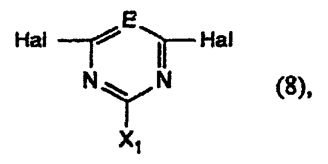 et un composé de formule
**T*-H (9),**
où U, E, W et X₁ possèdent chacun la signification donnée auparavant, Hal représente un atome d'halogène, de préférence le fluor ou le chlore, et T* possède la signification donnée à la revendication 1 pour T, dans une succession quelconque.

9. Procédé pour l'augmentation du facteur de protection solaire de matières fibreuses textiles **caractérisé en ce qu'**on applique un ou plusieurs composés de formule (1) selon la revendication 1 dans une solution aqueuse ou aqueuse-organique à la matière fibreuse textile et ensuite on les fixe.

10. Utilisation de composés de formule (1) selon la revendication 1 pour l'augmentation du facteur de protection solaire de matières fibreuses textiles non teintes, teintes ou imprimées.

11. Utilisation de composés de formule (1) selon la revendication 1 pour la stabilisation photochimique de matières fibreuses textiles non teintes, teintes ou imprimées.

12. Composés de formule 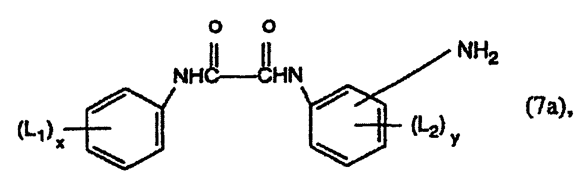 où (L₁)ₓ et (L₂)_{y} possèdent chacun la signification donnée.
